# EUROPÄISCHE PATENTSCHRIFT

(11) **EP 1 035 921 B1**
(45) Veröffentlichungstag und Bekanntmachung des Hinweises auf die Patenterteilung: **24.04.2002**
(21) Anmeldenummer: 98965760.6
(22) Anmeldetag: 04.12.1998
(51) Int. Cl.: B01L 3/00

(54) **ANALYTISCHES TESTELEMENT MIT KAPILLARKANAL**
ANALYTIC TEST ELEMENT WITH A CAPILLARY CANAL
ELEMENT D'ANALYSE A CANAL CAPILLAIRE

(30) Priorität: 04.12.1997 DE 19753847
(43) Veröffentlichungstag der Anmeldung: 20.09.2000
(73) Patentinhaber: Roche Diagnostics GmbH, 68305 Mannheim (DE)
(72) Erfinder: ZIMMER, Volker, D-69221 Dossenheim (DE); SCHWÖBEL, Wolfgang, D-68309 Mannheim (DE); MÖNCH, Ronald, D-68159 Mannheim (DE); LEICHNER, Wilhelm, D-68307 Mannheim (DE)
(86) Internationale Anmeldenummer: EP9807886
(87) Internationale Veröffentlichungsnummer: WO9929429

(56) Entgegenhaltungen:
- EP-A- 0 010 456
- EP-A- 0 330 517
- WO-A-98/22625
- US-A- 4 933 092
- US-A- 5 192 502
- US-A- 5 310 525

## Beschreibung

Die Erfindung betrifft ein analytisches Testelement zur Bestimmung eines Analyten in einer Flüssigkeit, enthaltend einen inerten Träger, ein Nachweiselement und einen zum kapillaren Flüssigkeitstransport befähigten Kanal, der eine Probenaufgabeöffnung an einem und eine Entlüftungsöffnung am anderen Ende des zum kapillaren Flüssigkeitstransports befähigten Kanals besitzt. Die Erfindung betrifft ebenfalls die Verwendung des besagten analytischen Testelements zur Bestimmung eines Analyten in einer Flüssigkeit sowie ein Verfahren zur Bestimmung eines Analyten in einer flüssigen Probe mit Hilfe des besagten analytischen Testelements.

Zur qualitativen oder quantitativen analytischen Bestimmung von Bestandteilen von Körperflüssigkeiten, insbesondere von Blut, werden oft sogenannte trägergebundene Tests verwendet. Bei diesen sind Reagenzien in entsprechenden Schichten eines festen Trägers eingebettet, der mit der Probe in Kontakt gebracht wird. Die Reaktion von flüssiger Probe und Reagenzien führt bei Anwesenheit eines Zielanalyten zu einem nachweisbaren Signal, insbesondere einem Farbumschlag, welcher visuell oder mit Hilfe eines Geräts, meist reflexionsphotometrisch, ausgewertet werden kann.

Testelemente oder Testträger sind häufig als Teststreifen ausgebildet, die im wesentlichen aus einer länglichen Tragschicht aus Kunststoffmaterial und darauf angebrachten Nachweisschichten als Testfeldern bestehen. Es sind jedoch auch Testträger bekannt, die als quadratische oder rechteckige Plättchen gestaltet sind.

Visuell oder reflexionsphotometrisch auszuwertende Testelemente für die klinische Diagnostik sind häufig so aufgebaut, daß die Probenauftragszone und die Detektionszone in einer vertikalen Achse übereinander angeordnet liegen. Diese Konstruktionsweise birgt eine Reihe von Problemen. Wenn der probenbeladene Teststreifen zur Vermessung in ein Gerät, beispielsweise ein Reflexionsphotometer, eingebracht werden muß, kann potentiell infektiöses Probenmaterial mit Geräteteilen in Berührung kommen und diese gegebenenfalls kontaminieren. Desweiteren ist, vor allem in den Fällen, in denen die Teststreifen von ungeschulten Personen benutzt werden, beispielsweise bei der Blutzuckerselbstkontrolle von Diabetikern, eine Volumendosierung nur schwer zu realisieren. Zudem benötigen herkömmliche Testelemente aufgrund ihres Aufbaus oftmals verhältnismäßig große Probenvolumina, um zuverlässige Messungen zu ermöglichen. Je mehr Probenvolumen benötigt wird, um so schmerzhafter kann dies für den Patienten, dessen Blut untersucht werden soll, sein. Es wird deshalb generell angestrebt, Teststreifen zur Verfügung zu stellen, die mit möglichst wenig Probenmaterial auskommen.

EP-B 0 138 152 behandelt eine Wegwerfküvette, die dazu geeignet ist, praktisch simultan Probenflüssigkeit mit Hilfe eines Kapillarspaltes in eine Probenkammer aufzunehmen und zu vermessen. Für spezifische Nachweisreaktionen können Reagenzien im Inneren des kapillaren Hohlraums vorgesehen sein. Der Hohlraum wird zumindest teilweise von einer semipermeablen Membran begrenzt. Die Reagenzien können beispielsweise durch Beschichtung der Wände oder durch Einbetten der Reagenzien in eine semipermeable Membran im Hohlraum untergebracht sein.

EP-A-0 287 883 beschreibt ein Testelement, das für die Volumendosierung einen kapillaren Zwischenraum zwischen Nachweisschicht und einem inerten Träger nutzt. Zur Befüllung des kapillaren Raumes wird das Testelement in die zu untersuchende Probe getaucht, was große Probenvolumina erforderlich macht, weshalb diese Form der Volumendosierung bevorzugt für die Untersuchung von im Überschuß vorhandenenem Probenmaterial, beispielsweise Urin, geeignet ist. Eine räumliche Trennung von Probenaufgabeort und Detektionsort findet nicht statt.

EP-B-0 034 049 beschäftigt sich mit einem Testelement, bei dem die Probe auf eine zentrale Probenaufgabestelle, beispielsweise eine Öffnung in einer Abdeckung, aufgegeben wird und mittels Kapillarkraft an mehrere räumlich von der Probenaufgabestelle getrennte Detektionszonen transportiert wird. Durch die zentrale Lage der Probenaufgabestelle ist mit einem Testelement gemäß EP-B-0 034 049 das Problem der Gerätehygiene, wie es oben beschrieben wurde, nicht gelöst.

Die Aufgabe der vorliegenden Erfindung bestand darin, die Nachteile des Standes der Technik zu beseitigen. Insbesondere sollte ein einfach zu handhabendes, selbständig volumendosierendes Testelement zu Verfügung gestellt werden, mit dem unter Verwendung minimaler Probenvolumina eine räumliche Trennung von Detektionszone und Probenaufgabestelle möglich ist. Zusätzlich sollte der Probentransport von der Probenaufgabe zum Detektionsbereich so schnell sein, daß die Analyse einer Probe zeitlich dadurch nicht limitiert wird. Desweiteren sollte durch einen einfachen Aufbau des Testelements eine kostengünstige, produktionstechnisch einfach zu realisierende Fertigung ermöglicht werden.

Dies wird durch den Gegenstand der Erfindung, wie er in den Patentansprüchen charakterisiert ist, erreicht.

Gegenstand der Erfindung ist ein analytisches Testelement zur Bestimmung eines Analyten in einer Flüssigkeit, enthaltend einen inerten Träger, ein Nachweiselement und einen zum kapillaren Flüssigkeitstransport befähigten Kanal, der eine Probenaufgabeöffnung an einem und eine Entlüftungsöffnung am anderen Ende des zum kapillaren Flüssigkeitstransports befähigten Kanals besitzt, dadurch gekennzeichnet, daß der zum kapillaren Flüssigkeitstransport befähigte Kanal zumindest teilweise vom Träger und dem Nachweiselement gebildet wird und in Richtung des kapillaren Transports von der Probenaufgabeöffnung zumindest bis zu der der Entlüftungsöffnung nächstgelegenen Kante des Nachweiselements reicht und daß sich eine Aussparung in einer den zum kapillaren Flüssigkeitstransport befähigten Kanal bildenden Fläche an der die Probenaufgabeöffnung bildende Kante des Testelements befindet, so daß die die Probenaufgabeöffnung bildende Kante des Testelements auf einer Seite zumindest teilweise unterbrochen ist und die der Aussparung gegenüberliegende Fläche frei liegt.

Da der zum kapillaren Flüssigkeitstransport befähigte Kanal in der Richtung des kapillaren Transports das Nachweiselement vollständig umfaßt, ist gewährleistet, daß eine inhomogene Benetzung des Nachweiselements mit Probe vermieden wird. Insbesondere ist die Schichtdicke der mit dem Nachweiselement in Kontakt stehenden Probenflüssigkeit durch die Höhe des kapillaraktiven Kanals über die gesamte Fläche des Nachweiselements, die den kapillaraktiven Kanal überdeckt, reproduzierbar vorgegeben. Dadurch wird eine weitgehend gleichmäßig räumlich verteilte Nachweisreaktion erzielt. Die Präzision und Reproduzierbarkeit der Messung wird somit erhöht.

Da für den bevorzugten Fall, daß der Kanal einen im wesentlichen rechteckigen Querschnitt aufweist, eine Dimension, beispielsweise die Höhe des Kanals, durch die physikalischen Grenzen der Kapillaraktivität vorgegeben ist, läßt sich das Volumen des kapillaren Kanals durch geeignete Wahl der beiden übrigen Dimensionen, beispielsweise Länge und Breite, einstellen. Die Höhe der Kapillare liegt beispielsweise für wäßrige Flüssigkeiten in der Größenordnung von 10 bis 500 µm, bevorzugt zwischen 20 und 300 µm, ganz bevorzugt zwischen 50 und 200 µm, da sonst keine Kapillaraktivität zu beobachten ist. Je nach gewünschtem Volumen kann die Breite dann mehrere mm, bevorzugt 1 bis 10 mm, ganz bevorzugt 1 bis 3 mm, und die Länge bis zu einigen cm, bevorzugt 0,5 bis 5 cm, ganz bevorzugt 1 bis 3 cm betragen.

Die Aussparung in einer den kapillaren Kanal bildenden Fläche an der Kante des Testelements, die die Probenaufgabeöffnung bildet, dient dazu, sicherzustellen, daß die Probenflüssigkeit in den kapillaren Kanal eintreten kann. Dies wird dadurch erreicht, daß der Probentropfen an der durch die Aussparung unterbrochenen Kante des Testelements, die der Probenaufgabeöffnung am nächsten liegt, direkt mit einer der Flächen in Kontakt gebracht werden kann, die in ihrer Verlängerung die innere Oberfläche der Kapillare bilden. Durch geeignete Wahl der Geometrie und Dimensionen der Aussparung wird erreicht, daß der Flüssigkeitstropfen unabhängig von der genauen Position der Dosierung mit sehr hoher Wahrscheinlichkeit mit der kapillaraktiven Zone in Kontakt kommt und bereitwillig in das Innere der Kapillare eingesaugt wird. Beispielsweise ist die Größe der frei liegenden Fläche so zu wählen, daß ein auf sie aufgebrachter Flüssigkeitstropfen zumindest an einer Stelle mit der kapillaraktiven Zone in Kontakt kommt. Beispielsweise ist eine Dimension der Aussparung. z. B. deren Breite, so zu wählen, daß der Durchmesser des Flüssigkeitstropfens geringfügig größer ist als die gewählte Dimension der Aussparung. Für eine Tropfen von 3 µl hat sich eine Breite der Aussparung von 1 mm als geeignet herausgestellt. Besonders bevorzugt wird das Einsaugen des Probentropfens in den kapillaren Kanal dadurch erreicht, daß die durch die Aussparung frei liegende Fläche hydrophiliert ist und zumindest in Richtung des kapillaren Transportkanals direkt an eine kapillaraktive Zone grenzt.

Hydrophile Oberflächen sind in diesem Zusammenhang wasseranziehende Flächen. Wäßrige Proben, darunter auch Blut, spreiten auf solchen Oberflächen gut. Solche Flächen sind unter anderem dadurch charakterisiert, daß an der Grenzfläche ein Wassertropfen auf ihnen einen spitzen Rand- oder Kontaktwinkel ausbildet. Im Gegensatz dazu wird auf hydrophoben, das heißt wasserabweisenden Oberflächen, an der Grenzfläche zwischen Wassertropfen und Oberfläche ein stumpfer Randwinkel ausgebildet.

Der Randwinkel als Resultat der Oberflächenspannungen der Prüfflüsigkeit und der zu untersuchenden Oberfläche ist als Maß für die Hydrophilie einer Oberfläche geeignet. Wasser hat beispielsweise eine Oberflächenspannung von 72 mN/m. Liegt der Wert der Oberflächenspannung der betrachteten Fläche weit, d. h. mehr als 20 mN/M, unter diesem Wert, so ist die Benetzung schlecht und der resultierende Randwinkel ist stumpf. Eine solche Fläche wird als hydrophob bezeichnet. Nähert sich die Oberflächenspannung dem Wert, der für Wasser gefunden wird, so ist die Benetzung gut und der Randwinkel wird spitz. Wird die Oberflächenspannung dagegen gleich oder größer dem für Wasser gefundenen Wert, so zerläuft der Tropfen und es findet Totalspreitung der Flüssigkeit statt. Ein Randwinkel ist dann nicht mehr zu messen. Flächen, die mit Wassertropfen einen spitzen Randwinkel bilden oder bei denen Totalspreitung eines Wassertropfens beobachtet wird, werden als hydrophil bezeichnet.

Die Bereitschaft einer Kapillare, eine Flüssigkeit aufzusaugen, geht mit der Benetzbarkeit der Kanaloberfläche mit der Flüssigkeit einher. Für wäßrige Proben bedeutet dies, daß eine Kapillare aus einem Material gefertigt werden sollte, dessen Oberflächenspannung nahe an 72 mN/m heranreicht oder diesen Wert übertrifft.

Ausreichend hydrophile Materialien zum Aufbau einer Kapillare, die schnell wäßrige Proben aufsaugt, sind beispielsweise Glas, Metall oder Keramik. Für den Einsatz in Testträgern sind diese Materialien jedoch ungeeignet, da sie einige gravierende Nachteile aufweisen, beispielsweise Bruchgefahr bei Glas oder Keramik, oder Veränderung der Oberflächeneigenschaften mit der Zeit bei zahlreichen Metallen. Üblicherweise werden deshalb zur Fertigung von Testelementen Kunstoffolien oder -formteile eingesetzt. Die verwendeten Kunststoffe übertreffen dabei in der Regel kaum eine Oberflächenspannung von 45 mN/m. Selbst mit den, relativ betrachtet, hydrophilsten Kunststoffen wie beipielsweise Polymethylmethacrylat (PMMA) oder Polyamid (PA) lassen sich - wenn überhaupt - nur sehr langsam saugende Kapillaren aufbauen. Kapillaren aus hydrophoben Kunststoffen wie beispielsweise Polystyrol (PS), Polypropylen (PP) oder Polyethylen (PE) saugen im wesentlichen keine wäßrigen Proben. Hieraus ergibt sich die Notwendigkeit, Kunststoffe für die Verwendung als Konstruktionsmaterial für Testelemente mit kapillaraktiven Kanälen hydrophil auszustatten, das heißt zu hydrophilieren.

In einer bevorzugten Ausführungsform des erfindungsgemäßen analytischen Testelements ist zumindest eine, besser jedoch zwei, ganz besonders bevorzugt zwei sich gegenüberliegende Flächen der die innere Oberfläche des zum kapillaren Flüssigkeitstransports befähigten Kanals bildenden Flächen, hydrophiliert. Ganz bevorzugt ist zumindest die der Aussparung gegenüberliegende, freiliegende Fläche hydrophiliert. Wird mehr als eine Fläche hydrophiliert, so können die Flächen entweder mit der gleichen oder mit unterschiedlichen Methoden hydrophil gemacht werden. Die Hydrophilierung ist vor allem dann notwendig, wenn die Materialien, die den kapillaraktiven Kanal bilden, insbesondere der Träger, selbst hydrophob oder nur sehr wenig hydrophil sind, beispielsweise weil sie aus unpolaren Kunststoffen bestehen. Unpolare Kunststoffe, wie zum Beispiel Polystyrol (PS), Polyethylen (PE), Polyethylenterephthalat (PET) oder Polyvinylchlorid (PVC), sind von Vorteil als Trägermaterialien, weil sie die zu untersuchenden Flüssigkeiten nicht absorbieren und damit das Probenvolumen effektiv von der Nachweisschicht genutzt werden kann. Durch die Hydrophilierung der Oberfläche des Kapillarkanals wird erreicht, daß eine polare, bevorzugt wäßrige Probenflüssigkeit bereitwillig in den kapillaren Kanal eintritt und dort rasch zum Nachweiselement bzw. zu der Stelle des Nachweiselements, an der die Detektion stattfindet, transportiert wird.

Idealerweise wird die Hydrophilierung der Oberfläche des kapillaren Kanals dadurch erreicht, daß zu seiner Fertigung ein hydrophiles Material eingesetzt wird, das jedoch die Probenflüssigkeit selbst nicht oder nicht wesentlich aufzusaugen vermag. Wo dies nicht möglich ist, kann die Hydrophilierung einer hydrophoben oder nur sehr wenig hydrophilen Oberfläche durch geeignete Beschichtung mit einer stabilen, gegenüber dem Probenmaterial inerten, hydrophilen Schicht erreicht werden, beispielsweise durch kovalente Bindung von photoreaktiv ausgerüsteten, hydrophilen Polymeren auf eine Kunststoffoberfläche, durch Aufbringen netzmittelhaltiger Schichten oder durch Beschichtung von Oberflächen mit Nanokompositen mittels Sol-Gel-Technologie. Darüberhinaus ist es möglich, durch thermische, physikalische oder chemische Behandlung der Oberfläche eine gesteigerte Hydrophilie zu erzielen.

Ganz besonders bevorzugt wird die Hydrophilierung durch die Verwendung von dünnen Schichten oxidierten Aluminiums erreicht. Diese Schichten werden entweder direkt auf die gewünschten Bauteile des Testelements aufgebracht, beispielsweise durch Vakuumbedampfen der Werkstücke mit metallischem Aluminium und anschließende Oxidation des Metalls, oder in Form von Metallfolien oder metallbeschichteten Kunststoffolien für den Testträgeraufbau verwendet, die ebenfalls zur Erzielung der erwünscheten Hydrophilie oxidiert werden müssen. Metallschichtdicken von 1 bis 500 nm sind dabei ausreichend. Die Metallschicht wird anschließend zu Bildung der oxidierten Form oxidiert, wobei sich neben der elektrochemischen, anodischen Oxidation vor allem die Oxidation in Gegenwart von Wasserdampf oder durch Kochen in Wasser als besonders geeignete Methoden herausgestellt haben. Die so erzielten Oxidschichten sind je nach Methode zwischen 0,1 und 500 nm, bevorzugt zwischen 10 und 100 nm dick. Größere Schichtdicken sowohl der Metallschicht als auch der Oxidschicht sind zwar prinzipiell praktisch realisierbar, zeigen aber keine weiteren vorteilhaften Wirkungen.

In einer bevorzugten Ausführungsform enthält das Nachweiselement des erfindungsgemäßen analytischen Testelements alle für die Nachweisreaktion des Zielananlyten in der Probe notwendigen Reagenzien und gegebenenfalls Hilfsstoffe. Das Nachweiselement kann auch nur Teile der Reagenzien oder Hilfsstoffe enthalten. Dem mit der Technik von analytischen Testelementen oder diagnostischen Testträgern vertrauten Fachmann sind solche Reagenzien und Hilfsmittel bestens bekannt. Für Analyten, die enzymatisch nachzuweisen sind, können beispielsweise Enzyme, Enzymsubstrate, Indikatoren, Puffersalze, inerte Füllstoffe und dergleichen mehr im Nachweiselement enthalten sein. Das Nachweiselement kann aus einer oder mehreren Schichten aufgebaut sein und gegebenenfalls einen inerten Träger, bevorzugt auf der Seite des Nachweiselements, die nicht mit der Probe in Kontakt gebracht wird, enthalten. Für den besonders bevorzugten Fall, daß die Nachweisreaktion zu einer beobachtbaren Farbveränderung führt, worunter in diesem Zusammenhang entweder die Änderung einer Farbe, das Entstehen einer Farbe oder das Verschwinden von Farbe verstanden werden soll, ist sicherzustellen, daß der Träger durch geeignete Maßnahmen eine visuelle oder optische Beobachtung der Nachweisreaktion zuläßt. Dazu kann das Trägermaterial des Nachweiselements selbst durchsichtig sein, beispielsweise eine durchsichtige Kunststoffolie, wie beispielsweise Polycarbonatfolie, oder auf der Detektionsseite eine durchsichtige Aussparung besitzen. Neben Nachweisreaktionen, die zu Farbveränderungen führen, sind dem Fachmann auch andere Nachweisprinzipien bekannt, die mit dem beschriebenen Testelement realisiert werden können, beispielsweise elektrochemische Sensoren.

Für das Nachweiselement ist es erforderlich, solche Materialien einzusetzen, die in der Lage sind, die zu untersuchende Flüssigkeit mit darin enthaltenen Inhaltsstoffen aufzunehmen. Dies sind sogenannte saugfähige Materialien, wie beispielsweise Vliese, Gewebe, Gewirke oder poröse Kunststoffmaterialien, die als Schichtmaterialien verwendet werden können. Die dafür in Frage kommenden Materialien müssen Reagenzien tragen können, die für den Nachweis des zu bestimmenden Analyts erforderlich sind.

Bevorzugte Materialien für das Nachweiselement sind Papiere oder poröse Kunststoffmaterialien, wie Membranen. Als poröse Membranmaterialien sind Polyamid-, Polyvinylidendifluorid-, Polyethersulfon- oder Polysulfonmembranen ganz besonders bevorzugt. Die Reagenzien zur Bestimmung des nachzuweisenden Analyts sind in der Regel durch Imprägnierung in die vorstehend genannten Materialien eingebracht worden.

Für das Nachweiselement kommen besonders bevorzugt sogenannte offene Filme in Frage, wie sie beispielsweise in EP-B-0 016 387 beschrieben sind. Hierfür werden einer wäßrigen Dispersion von filmbildenden organischen Kunststoffen Feststoffe als feine, unlösliche, organische oder anorganische Partikel zugegeben und die für die Nachweisreaktion erforderlichen Reagenzien zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wäßrigen Dispersionen. Die Dispersionen lassen sich auf einer Unterlage zu einer gleichmäßigen Schicht verstreichen, die nach dem Trocknen einen wasserfesten Film ergibt. Die trocknen Filme haben eine Dicke von 10 µm bis 500 µm, vorzugsweise von 30 bis 200 µm. Der Film kann mit der Unterlage als Träger zusammen verwendet werden oder für die Nachweisreaktion auf einen anderen Träger aufgebracht werden. Obwohl die für die Nachweisreaktion erforderlichen Reagenzien normalerweise in die zur Herstellung der offenen Filme verwendete Dispersion gegeben werden, kann es auch vorteilhaft sein, wenn der gebildete Film nach seiner Herstellung mit den Reagenzien imprägniert wird. Auch eine Vorimprägnierung der Füllstoffe mit den Reagenzien ist möglich. Welche Reagenzien zur Bestimmung eines bestimmten Analyts eingesetzt werden können sind dem Fachmann bekannt. Dies muß hier nicht näher ausgeführt werden.

Das Nachweiselement kann zudem über Bestandteile verfügen, die einen Ausschluß störender Probenanteile von der Nachweisreaktion erlauben und somit als Filter, beispeilsweise für partikuläre Probenbestandteile wie Blutzellen, wirken. Für visuelle oder optische Detektionsverfahren ist beispielsweise bei der Analyse von Blutproben der rote Blutfarbstoff Hämoglobin, der in den roten Blutkörperchen (Erythrozyten) enthalten ist, störend. Zweckmäßigerweise werden diese störenden Komponenten vor der eigentlichen Detektionsreaktion von der Probe, beispielsweise Vollblut, abgetrennt. Dies kann durch Probenaufbereitung vor dem Applizieren der Probe auf das Testelement geschehen, wie zum Beispiel durch Zentrifugieren von Vollblut und anschließender Serum- oder Plasmagewinnung. Bequemer und auch für den Laien einfacher ist es, wenn das Testelement diesen Trennungsschritt durch geeignete Konstruktion selbst durchführt. Dem Fachmann sind Mittel aus der Teststreifentechnologie bekannt, die einen zuverlässigen Ausschluß von Erythrozyten gewährleisten. Beispielsweise seien genannt die Verwendung von semipermeablen Membranen oder Glasfaservliesen, wie sie beispielsweise aus EP-B-0 045 476 bekannt sind, zur Abtrennung roter Blutkörperchen.

Als besonders bevorzugt hat sich erwiesen, für das erfindungsgemäße Testelement ein Nachweiselement bestehend aus zwei Filmschichten auf einer transparenten Folie zu verwenden. Wesentlich ist, daß die auf der transparenten Folie liegende erste Schicht bedeutend weniger lichtstreuend ist als die darüberliegende zweite Schicht. Solche Nachweiselemente sind beispielsweise aus der deutschen Patentanmeldung Nr. P 196 29 656.0 bekannt.

Während die erste Schicht ein Quellmittel, wie zum Beispiel Methylvinylether-Maleinsäure Copolymer, und gegebenenfalls einen schwach lichtstreuenden Füllstoff enthält, benötigt die zweite Schicht ein Quellmittel und in jedem Fall wenigstens ein stark lichtsstreuendes Pigment und kann daneben auch nicht-poröse Füllstoffe sowie poröse Füllstoffe, wie Kieselgur in geringen Mengen enthalten, ohne dadurch für Erythrozyten durchlässig zu werden.

Da die schwach lichtstreuenden Füllstoffe und die stark lichtstreuenden Pigmente wesentlich für die optischen Eigenschaften der Filmschichten verantwortlich sind, besitzen die erste und die zweite Filmschicht unterschiedliche Füllstoffe und Pigmente. Die erste Filmschicht soll entweder keine oder solche Füllstoffe enthalten, deren Brechungsindex nahe beim Brechungsindex von Wasser liegt, beispielsweise Siliziumdioxid, Silikate und Aluminiumsilikate. Die mittlere Korngröße besonders bevorzugter Füllstoffteilchen beträgt etwa 0,06 µm. Die zweite Schicht soll zweckmäßigerweise sehr stark lichtstreuend sein. Idealerweise liegt der Brechungsindex der Pigmente in der zweiten Filmschicht mindestens bei 2,5. Daher wird vorzugsweise Titandioxid eingesetzt. Teilchen mit einem mittleren Durchmesser von etwa 0,2 bis 0,8 µm haben sich als besonders vorteilhaft erwiesen.

Weiterhin hat es sich als bevorzugt herausgestellt, daß der zum kapillaren Transport befähigte Kanal außer vom inerten Träger und dem Nachweiselement zusätzlich von einer Abdeckung gebildet wird, die bevorzugt neben dem Nachweiselement und wie dieses auf der dem Träger gegenüberliegenden Seite des Kanals liegt. Die Abdeckung kann in ihren Eigenschaften, wie zum Beispiel Material und Beschichtung, ähnlich oder gleich dem Träger sein. Sie ersetzt auf einem Teilstück, bevorzugt auf der der Probenaufgabeöffnung zugewandten Seite, der kapillaren Transportstrecke das Nachweiselement. Da dieses gewöhnlich wertvolle Reagenzien, wie beispielsweise Enzyme, enthält und aufgrund seines oftmals komplexen Aufbaus in der Herstellung um ein Vielfaches teurer ist als für die Abdeckung geeignete Materialien, ergibt sich durch diese Maßnahme eine deutliche Verringerung der Material- und Produktionskosten. Dies wirkt sich vor allem bei langen kapillaren Transportstrecken aus, worunter hier Strecken von mehr als 5 mm zu verstehen sind. Zudem kann durch diese Maßnahme erreicht werden, daß bei Testelementen, bei denen in einem räumlich genau definierten Bereich die Nachweisreaktion im Nachweiselement detektiert wird, zum Beispiel bei optischer Detektion in einem Gerät, und bei denen eine Trennung von Probenaufgabezone und Detektionszone, beispielsweise aus Gründen der Gerätehygiene, angestrebt ist, ein beschleunigter Probentransport von der Probenaufgabeöffnung im Testelement zur Detektionsstelle im Nachweiselement erfolgt, so daß der Transport der Probe im kapillaren Kanal von der Probenaufgabezone zum Detektionsbereich so schnell ist, daß die Analyse einer Probe zeitlich dadurch nicht limitiert wird. Außerdem wird durch eine solche Anordnung eine bequemere Anwendung für den Benutzer erreicht.

Die Montage von Abdeckung und Nachweiselement hat so zu erfolgen, daß im fertigen Testelement beide Stoß an Stoß nebeneinander zu liegen kommen, so daß der Flüssigkeitstransport in der Kapillare an ihrer Berührungsstelle nicht unterbrochen wird, beispielsweise durch ungünstige Veränderung des Kapillarenquerschnitts, worunter auch die Unterbrechung einer geschlossenen Begrenzungsfläche der Kapillare verstanden wird. Nachweiselement und Abdeckung sind diesem Zweck entsprechend in ihren Dimensionen aneinander anzupassen. Ist eine ausreichend enge Montage der beiden Komponenten nicht möglich, kann durch nachträgliches Abdichten der kapillare Schluß erzielt werden.

Überraschenderweise wurde gefunden, daß für eine ganz besonders bevorzugte Ausführungsform des erfindungsgemäßen Testträgers auf der dem zum kapillaren Flüssigkeitstransport befähigten Kanal hin zugewandten Seite der Abdeckung eine flexible, inerte Folie angebracht werden kann, die über die gesamte Länge der Abdeckung reicht, den kapillaren Kanal auf der gesamten Breite bedeckt und die zumindest teilweise zwischen den sich gegenüberliegenden Flächen der Abdeckung und des Nachweiselements eingeschlossen ist, so daß der kapillare Flüssigkeitstransport an der Berührungsstelle von Nachweiselement und Abdeckung nicht abreißt. Die Folie kann vom Material und gegebenenfalls ihrer hydrophilierenden Beschichtung her weitgehend dem entsprechen, was weiter oben für Träger und Abdeckung beschrieben wurde. Nachweiselement und Abdeckung sind auch bei dieser ganz besonders bevorzugten Variante möglichst eng montiert.

Weiterhin kann das erfindungsgemäße Testelement in einer bevorzugten Ausführungsform zwischen dem Träger auf der einen Seite des Kapillarkanals und dem Nachweiselement und gegebenenfalls der Abdeckung auf der gegenüberliegenden Seite eine Zwischenschicht enthalten, die ebenfalls wie die vorgenannten Komponenten an der Ausbildung des kapillaraktiven Kanals beteiligt ist. Ganz besonders bevorzugt hat die Zwischenschicht eine Länge in Richtung des kapillaren Transports, die zumindest der Länge des Kanals entspricht. Zweckmäßigerweise wird die Zwischenschicht so gestaltet, daß sie Breite und gegebenenfalls Höhe des zum kapillaraktiven Transports befähigten Kanals bestimmt. Bevorzugt hat die Zwischenschicht dazu eine Aussparung, beispielsweise eine Ausstanzung, die den Dimensionen Breite und Höhe des kapillaraktiven Kanals entspricht. Besonders bevorzugt ist die Länge der Aussparung geringfügig größer als die Länge des kapillaraktiven Kanals, um somit eine Entlüftungsöffnung zu schaffen. Die Zwischenschicht kann prinzipiell aus den selben Materialien und gegebenenfalls mit den selben Beschichtungen gefertigt werden, aus denen Träger und/oder Abdeckung bestehen. Als besonders bevorzugt hat es sich jedoch erwiesen, die Zwischenschicht aus doppelseitig klebendem Band oder Streifen zu fertigen, da die Zwischenschicht dann auch die Funktion übernehmen kann, Träger und Nachweiselement und gegebenenfalls Abeckung miteinander zu verbinden. Diese Verbindung kann gegebenenfalls auch auf andere Weise, beispielsweise durch Verschweißen, Heißsiegeln, beispielsweise mit Polyethylen, Verklebung mit härtendem Kaltkleber oder Schmelzkleber, oder Clipsen erreicht werden.

Neben den bereits genannten Vorteilen des erfindungsgemäßen Testelements weist es weitere Vorzüge auf. Durch die räumliche Trennung von Probenaufgabeort und Signaldetektion in Verbindung mit der Probenvolumendosierung wird eine hygienische Handhabung des Probenmaterials erreicht. Vor allem bei optischer Detektion, beispielsweise mithilfe eines Reflexionsphotometers, wird eine Kontamination des Gerätes weitestgehend ausgeschlossen, da die Probe beispielsweise auf ein aus dem Gerät herausragendes Testelement aufgegeben werden kann, dabei die zur Bestimmung des Analyten erforderliche Menge an Probe in den kapillaren Kanal eingesaugt wird und selbständig ohne weitere Maßnahmen zu der im Geräteinneren gelegenen Detektionszone des Testelements transportiert wird.

Desweiteren benötigt das erfindungsgemäße Testelement in einer ganz besonders bevorzugten Ausführungsform bedeutend weniger Probenmaterial als herkömmliche Testelemente. Während letztere oftmals über 12 µl Probenflüssigkeit benötigen, wird das erforderliche Probenmindestvolumen bei dem erfindungsgemäßen Testelement auf deutlich unter 10 µl, bevorzugt unter 5 µl, besonders bevorzugt auf 3 bis 4 µl Probe gesenkt. Dies wird durch die Optimierung des Probenflusses genau an den Bestimmungsort, sowie durch die definierte Schichtdicke des Probenmaterials unter dem Nachweisfeld erreicht. Insbesondere für den Fall, daß die Probe Blut ist, kann dadurch für die zu untersuchend Person die Probengewinnung einfacher und vor allem mit weniger Schmerz verbunden sein.

Ein weiterer Gegenstand der Erfindung ist die Verwendung eines erfindungsgemäßen analytischen Testelements zur Bestimmung eines Analyten in einer Flüssigkeit.

Außerdem ist Gegenstand der Erfindung ein Verfahren zur Bestimmung eines Analyten in einer flüssigen Probe, insbesondere einer Körperflüssigkeit wie Blut, Plasma, Serum, Urin, Speichel, Schweiß etc., mit Hilfe eines erfindungsgemäßen analytischen Testelements. Dabei wird zunächst die flüssige Probe mit der durch die Aussparung unterbrochene Kante der Probenaufgabeöffnung mit dem Testelement kontaktiert. Durch Kapillarkräfte wird die Probenflüssigkeit in den zum kapillaren Flüssigkeitstransport befähigten Kanal transportiert. Die Probe benetzt dabei das Nachweiselement auf der dem Kanal zugewandten Oberfläche und dringt in dieses ein. Gegebenenfalls geht die Probe mit den im Nachweiselement enthaltenen Reagenzien eine analytspezifische visuell oder apparativ-optisch, bevorzugt reflexionsphotometrisch beobachtbare Nachweisreaktion ein, so daß auf die Anwesenheit und gegebenenfalls die Menge des zu bestimmenden Analyten rückgeschlossen werden kann.

Die Erfindung wird durch die Figuren 1 bis 6 und die nachfolgenden Beispiele näher erläutert.

Figur 1 zeigt eine besonders bevorzugte Ausführungsform des erfindungsgemäßen Testelements. In Figur 1A ist eine schematische Aufsicht auf das erfindungsgemäße Testelement zu sehen, die Figuren 1B bis 1F zeigen jeweils Querschnittsdarstellungen entlang der Linien A-A', B-B', C-C', D-D' bzw. E-E'.

Figur 2 zeigt eine weitere besonders bevorzugte Ausführungsform des erfindungsgemäßen Testelements. In Figur 2A ist eine schematische Aufsicht auf das erfindungsgemäße Testelement abgebildet. Die Figuren 2B bis 2F zeigen jeweils Querschnitte entlang der Linien A-A', B-B', C-C', D-D' bzw. E-E'.

Figur 3 zeigt ebenfalls eine besonders bevorzugte Ausführungsform des erfindungsgemäßen Testträgers. Figur 3A ist eine Aufsicht auf das Testelement. Die Figuren 3B bis 3F zeigen jeweils Querschnitte entlang der Achsen A-A', B-B', C-C', D-D' bzw. E-E'.

Figur 4 stellt wiederum eine besonders bevorzugte Ausführungsform des erfindungsgemäßen Testträgers dar. Figur 4A zeigt schematisch eine Aufsicht auf das Testelement. Die Figuren 4B bis 4D sind Querschnittsdarstellungen entlang der Linien C-C', D-D' bzw. E-E'.

Figur 5 zeigt eine ganz besonders bevorzugte Ausführungsform des erfindungsgemäßen Testelements. In Figur 5A ist die schematische Aufsicht auf das Testelement zu sehen. Die Figuren 5B bis 5G zeigen jeweils Querschnitte entlang der Linien A-A' (5B), B-B' (5C), C-C' (5D und 5G), D-D' (5E) bzw. E-E' (5F).

Figur 6 zeigt eine perspektivische Detailvergrößerung des Probenaufgabebereichs des erfindungsgemäßen Testträgers.

Die Ziffern in den Figuren bedeuten:
- 1: Träger
- 2: Nachweiselement
- 3: Kapillarer Kanal
- 4: Probenaufgabeöffnung
- 5: Aussparung für Probenaufgabe
- 6: Entlüftungsöffnung
- 7: Abdeckung
- 8: Spaltabdeckfolie
- 9: Zwischenschicht
- 10: Stützfolie

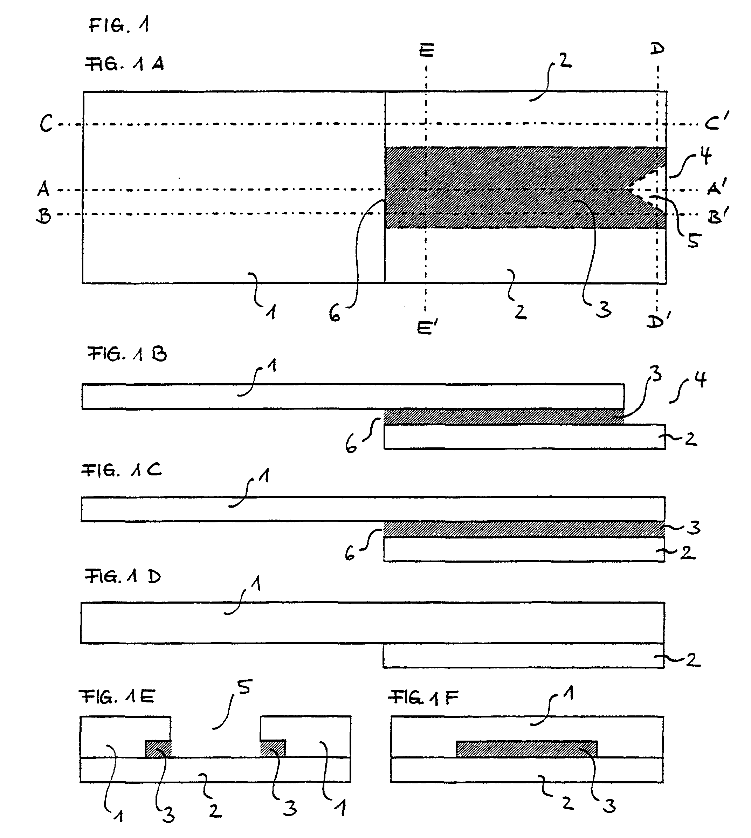

In Figur 1 ist eine besonders bevorzugte Ausführungsform des erfindungsgemäßen Testelements schematisch in verschiedenen Ansichten dargestellt (Figur 1A bis 1F). Die gezeigten Ansichten sollen einen plastischen Eindruck des erfindungsgemäßen Testelements liefern. Das Testelement besteht aus einem Träger (1), der so geformt ist, daß er dort, wo er mit dem Nachweiselement (2) bedeckt ist, zusammen mit diesem einen kapillaren Kanal (3) bildet. Beispielsweise kann eine Vertiefung in den Träger geprägt oder gefräst sein. In der gezeigten Ausführungsform ist auf der Probenaufgabeöffnung (4) des Testelements im Träger (1) eine Aussparung (5) vorgesehen, die es ermöglicht, bei der Probenaufgabe den Flüssigkeitstropfen unmittelbar mit der kapillaraktive Zone (3) in Kontakt zu bringen. An der der Probenaufgabeöffnung (4) gegenüberliegenden Seite des kapillaren Kanals (3) befindet sich eine Entlüftungsöffnung (6), die das Entweichen von Luft bei der Befüllung des Kapillarkanals mit Probenflüssigkeit erlaubt.

Die Kapillarzone (3) reicht von der Probenaufgabeöffnung (4) bis zum entgegengesetzten Ende des Nachweiselements (2) und gewährleistet somit eine homogene Probenverteilung über das Nachweiselement (2). Probenaufgabeöffnung (4) und Entlüftungsöffnung (6) begrenzen den kapillaraktiven Bereich (3) in Richtung des kapillaren Transports.

Bei der Verwendung des gezeigten Testelements wird das Testelement mit der Probenaufgabeöffnung (4) beispielsweise mit einem sich an der Fingerkuppe befindlichenden Blutstropfen in Kontakt gebracht. Dabei kommt der Blutstropfen durch die Aussparung (5) im Träger (1) mit der freiliegenden Fläche, die gegebenenfalls hydrophiliert ist, und gleichzeitig dem kapillaren Kanal (3) in Kontakt. Letzterer füllt sich solange mit Probe, bis er von der Probenaufgabeöffnung (4) bis zur Entlüftungsöffnung (6) gefüllt ist. Danach wird der Testträger vom Patientenfinger entfernt, wodurch gewährleistet ist, daß lediglich die sich im Kapillarkanal (3) befindliche Probe für das Nachweiselement (2) verfügbar ist.

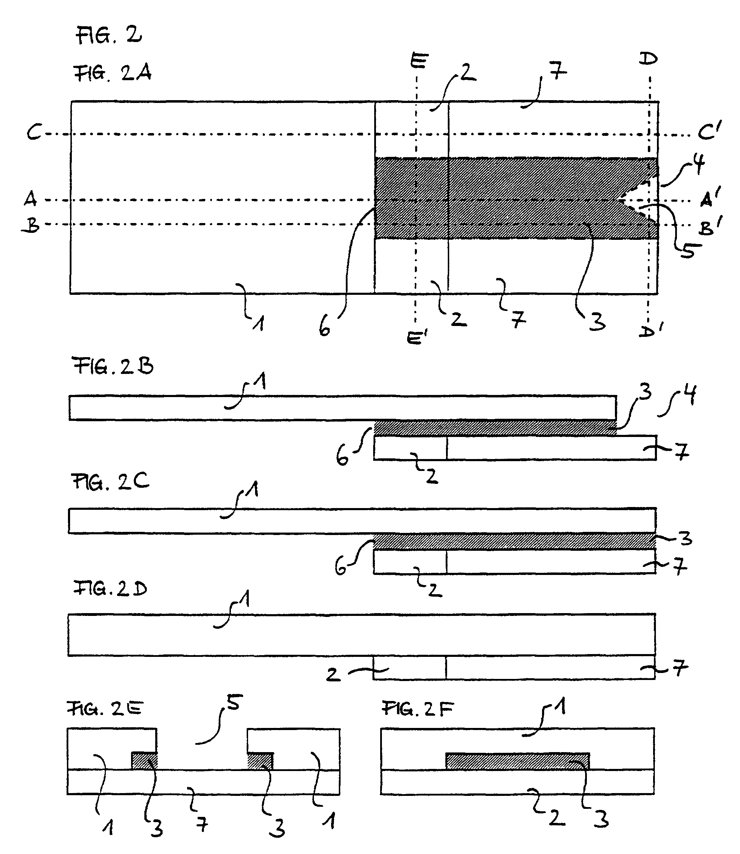

In Figur 2 ist eine weitere besonders bevorzugte Ausführungsform als Alternative zu dem in Figur 1 dargestellten Testelement abgebildet. Die Teilansichten Figur 2A bis 2F sollen wiederum einen räumlichen Eindruck des erfindungsgemäßen Testelements vermitteln. Das gezeigte Testelement enthält einen zum kapillaren Flüssigkeitstransport befähigten Kanal (3), der von einem inerten Träger (1), dem Nachweiselement (2) und einer Abdeckung (7) gebildet wird. Abdeckung (7) und Nachweiselement (2) sind so Stoß an Stoß nebeneinander montiert, daß der kapillare Kanal (3) ununterbrochen von der Probenaufgabeöffnung (4) bis zur Entlüftungsöffnung (6) reicht. Das gezeigte Testelement enthält widerum eine Aussparung (5), die das Eindringen der Probenflüssigkeit in den Kapillarkanal erleichtert.

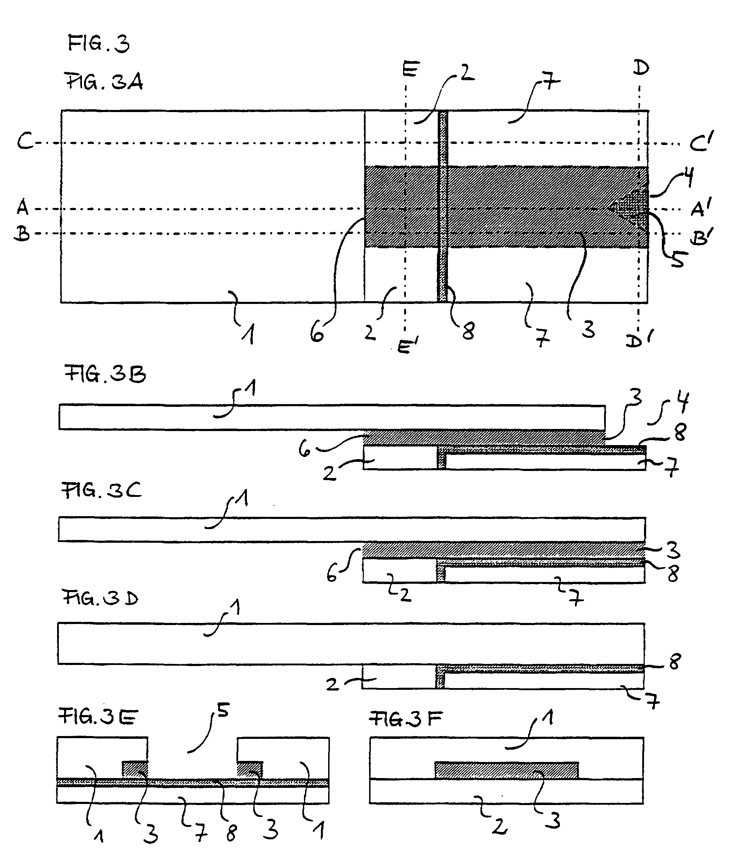

In Figur 3 wird schematisch anhand unterschiedlicher Ansichten (Figur 3A bis 3F) gezeigt, wie durch die Verwendung einer Spaltabdeckfolie (8) das Abreißen des kapillaraktiven Bereichs (3) an der Berührungsstelle von Nachweiselement (2) und Abdeckung (7) zuverlässig vermieden werden kann. Die Spaltabdeckfolie (8) kann zudem auf der dem Kapillarkanal (3) zugewandten Seite mit einer hydrophilen Oberfläche ausgestattet sein, die für den kapillaren Transport eines Probenflüssigkeitstropfens von der Probenaufgabeöffnung (4) zur Entlüftungsöffnung (6) förderlich ist. Insbesondere ist eine solche Hydrophilierung im Bereich der Aussparung (5) im Träger (1) von Vorteil, da sie das Eindringen des Probenmaterials in den kapillaren Kanal beschleunigt.

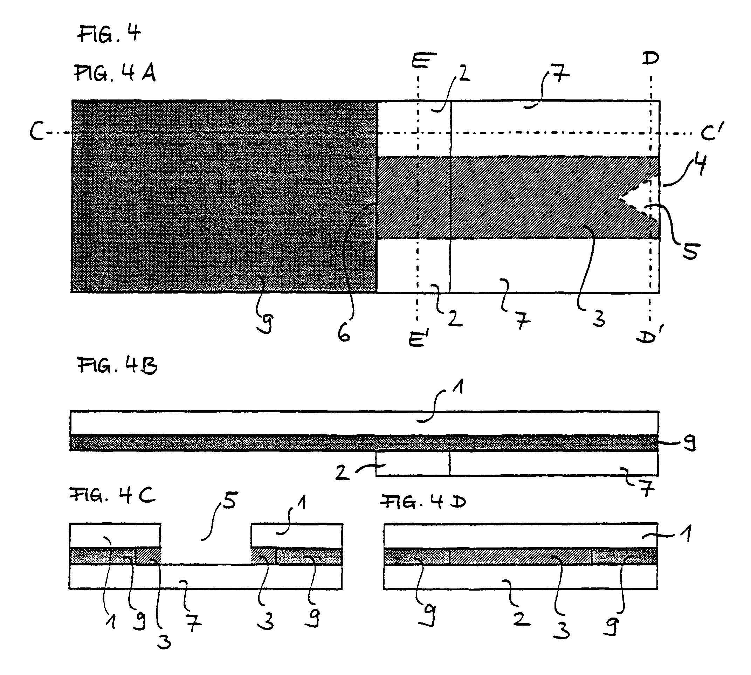

Im Gegensatz zu den in den Figuren 1 bis 3 gezeigten besonders bevorzugten Ausführungsformen des erfindungsgemäßen Testträgers wird bei dem in Figur 4 abgebildeten Testelement, das ebenfalls eine besonders bevorzugte Ausführungsform des erfindungsgemäßen Gegenstandes ist, die Geometrie des kapillaren Kanals (3) nicht von der Form des Trägers (1) bestimmt, sondern maßgeblich von einer Zwischenschicht (9) geprägt. Die Figuren 4A bis 4D sollen wiederum einen plastischen Eindruck des Testträgeraufbaus vermitteln. Die Zwischenschicht (9) kann aus einem doppelseitig klebenden Band gefertigt sein, das neben der Bestimmung der Kapillarkanalgeometrie ebenfalls das Verbinden der übrigen, an der Bildung der kapillaraktiven Zone (3) beteiligten Komponenten Träger (1), Abdeckung (7) und Nachweiselement (2) zum Zweck hat. Abdeckung (7) und Nachweiselement (2) sind bei dem gezeigten Testelement wiederum so eng Stoß an Stoß montiert, daß der Kapillarkanal (3) ununterbrochen von der Aussparung (5) an der Probenaufgabeöffnung (4) bis zur Entlüftungsöffnung (6) reicht.

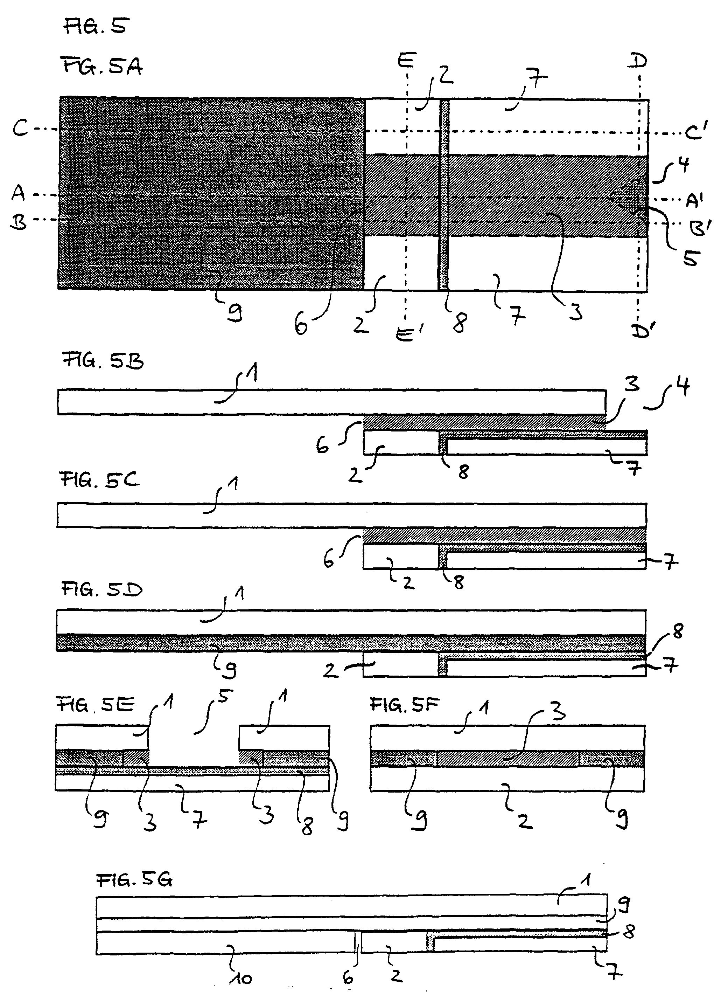

Das in Figur 5A bis 5F in unterschiedlichen Ansichten abgebildete Testelement stellt eine ganz besonders bevorzugte Ausführungsform des Erfindungsgegenstandes dar. Es verbindet in sich sämtliche Komponenten und damit andere Vorzüge der Testelemente, die in den Figuren 1 bis 4 abgebildet sind.

Auf einem Träger (1) ist eine Zwischenschicht (9) in Form eines doppelseitig klebenden Bandes angebracht. Die Zwischenschicht (9) besitzt im Bereich des kapillaren Kanals (3) eine Aussparung, die Länge und Breite des Kanals (3) bestimmt. Seine Höhe ist durch die Dicke der Zwischenschicht (9) vorgegeben. Auf der dem Träger (1) gegenüberliegenden Seite des Kapillarkanals (3) befindet sich neben dem Nachweiselement (3) eine Abdeckung (7). Um kapillaren Schluß zu gewährleisten ist eine Spaltabdeckfolie (8) vorgesehen. Diese kann hydrophiliert sein, so daß sie einen schnellen Probentransport von der Probenaufgabeöffnung (4) zur Entlüftungsöffnung (6) ermöglicht, die das entgegengesetzte Ende des kapillaren Kanals markiert. Die Hydrophilierung hat daneben den Vorteil, daß im Bereich der Aussparung (5) ein Probenflüssigkeitstropfen direkt auf eine hydrophile Fläche aufgebracht werden kann, die an mehreren Begrenzungsseiten von kapillaraktiver Zone (3) umgeben ist. Dies führt zu einem raschen Eindringen des Flüssigkeitstropfens in das Testelement.

In Figur 5 G ist gezeigt, wie die Zwischenschicht (9) durch eine Stützfolie (10) abgedeckt werden kann, um freiliegende Klebebandbereiche zu bedecken. Die Entlüftungsöffnung (6) darf dabei jedoch nicht überdeckt werden.

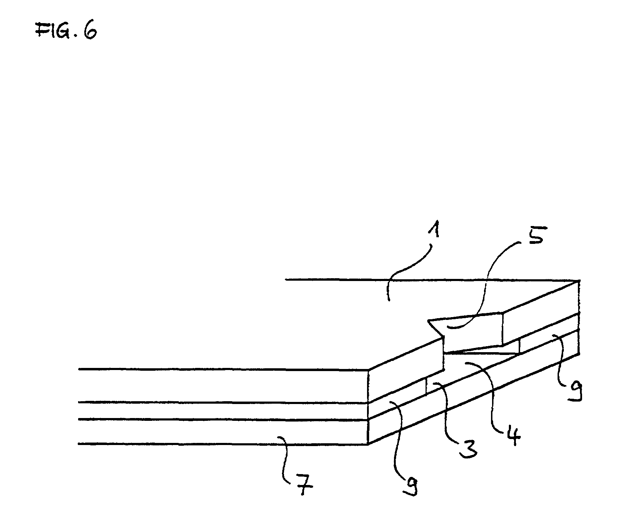

In Figur 6 ist schließlich eine Detailvergrößerung in perspektivischer Ansicht des Probenaufgabebereiches einer besonders bevorzugten Ausführungsform des erfindungsgemäßen Testelements abgebildet. Die Aussparung (5) im Träger (1) erleichtert das Eindringen einer Probenflüssigkeit von der Probenaufgabeöffnung (4) in die kapillaraktive Zone (3), die im vorliegenden Fall von Träger (1), Zwischenschicht (9) und Abdeckung (7) gebildet wird.

### Beispiel 1

### Herstellung des erfindungsgemäßen analytischen Testelements

Auf eine mit einer 30 nm dicken Schicht aus Aluminium, das mit Wasserdampf vollständig oxidiert wurde, beschichtete, 350 µm dicke Folie aus Polyethylenterephthalat (Melinex®, ICI, Frankfurt am Main, Deutschland) wird ein doppelseitiges Klebeband der Dicke 100 µm geklebt. Die Folie hat eine Länge von 25 mm und ist 5 mm breit. An einer der kurzen Seiten befindet sich eine zentrale, kerbenförmige Aussparung von 1 mm Breite und 2 mm Länge. Das Klebeband besitzt eine Ausstanzung von 2 mm Breite und mehr als 15 mm Länge, die die Dimensionen des Kapillarkanals definieren. Die Länge der Ausstanzung ist geringfügig größer zu wählen als die gewünschte Länge des kapillaraktiven Kanals, die durch dessen Abdeckung bestimmt wird, um eine Entlüftung des Kanals während des Befüllens mit Probenflüssigkeit zu gewährleisten. Auf das Klebeband wird auf der Seite, an der die Entlüftung vorgesehen ist, in 1 mm Abstand vom Ende der Ausstanzung ein 3 mm langer und 5 mm breiter Nachweisfilm geklebt. Als Nachweisfilm wird ein Film verwendet, wie er aus der deutschen Patentanmeldung Nr. P 196 29 656.0 bekannt ist. Der Nachweisfilm ist spezifisch für den Nachweis von Glucose. Auf den noch offen liegenden Bereich des Klebebandes zwischen kerbenförmiger Aussparung und Nachweisfilm wird eine 12 mm lange und 5 mm breite Abdeckschicht aufgeklebt, so daß Abdeckschicht und Nachweisfilm Stoß an Stoß zu liegen kommen. Die Abdeckschicht besteht aus einer 150 µm dicken, einseitig mit Klebstoff versehenen Polyethylenterephthalat-Folie, auf die auf der zum Kapillarkanal hingewandten Seite eine mit oxidiertem Aluminium der Dicke 30 nm beschichtete, 6 µm dicke Polyethylenterephthalat-Folie geklebt ist ( beide: Hostaphan®, Hoechst, Frankfurt am Main, Deutschland). Die dünnere Folie steht dabei an der zum Nachweisfilm gewandten Seite ca. 500 µm über die dickere Folie über. Bei der Montage der Abdeckschicht auf das Klebeband ist darauf zu achten, daß das überstehende Ende der dünneren Folie zwischen dem Nachweiselement und der dickeren Folie der Abdeckschicht zu liegen kommt. Um noch frei liegende Klebebandbereiche abzudecken werden diese mit einer 175 µm dicken Melinex®-Folie abgedeckt, ohne dabei jedoch funktionale Bereiche abzudecken.

Das so erhaltene Testelement hat eine kapillaren Kanal von 15 mm Länge, 2 mm Breite und 0,1 mm Höhe. Der Kanal kann 3 µl Probenflüssigkeit aufnehmen. Der Nachweisfilm wird auf einer Fläche von 3 mm × 2 mm von der Probe benetzt.

### Beispiel 2

### Messung der Blutglukosekonzentration mit Hilfe des Testelements aus Beispiel 1

Das Testelement aus Beispiel 1 wird mit der Probenaufgabeseite auf einen Probenflüssigkeitstropfen aufgesetzt. Die Kapillare des Testelements füllt sich innerhalb von 2 s selbständig mit Probe. Ist Glucose in der Probe vorhanden wird nach wenigen Sekunden eine Farbentwicklung im Nachweisfilm sichtbar. Nach ca. 30 bis 35 s ist der Endpunkt der Reaktion erreicht. Die erhaltene Farbe kann mit der Glucosekonzentration der Probe korreliert werden und wird entweder visuell oder reflexionsfotometrisch ausgewertet.

## Patentansprüche

1. Analytisches Testelement zur Bestimmung eines Analyten in einer Flüssigkeit, enthaltend einen inerten Träger, ein Nachweiselement und einen zum kapillaren Flüssigkeitstransport befähigten Kanal, der eine Probenaufgabeöffnung an einem und eine Entlüftungsöffnung am anderen Ende des zum kapillaren Flüssigkeitstransports befähigten Kanals besitzt, wobei der zum kapillaren Flüssigkeitstransport befähigte Kanal zumindest teilweise vom Träger und dem Nachweiselement gebildet wird und in Richtung des kapillaren Transports von der Probenaufgabeöffnung zumindest bis zu der der Entlüftungsöffnung nächstgelegenen Kante des Nachweiselements reicht, ***dadurch gekennzeichnet, daß*** sich eine kerbenförmige Aussparung in einer den zum kapillaren Flüssigkeitstransport befähigten Kanal bildenden Fläche an der die Probenaufgabeöffnung bildenden Kante des Testelements befindet, so daß die die Probenaufgabeöffnung bildende Kante des Testelements auf einer Seite teilweise unterbrochen ist und die der Aussparung gegenüberliegende Fläche frei liegt.

2. Analytisches Testelement gemäß Anspruch 1, ***dadurch gekennzeichnet, daß*** zumindest eine der die innere Oberfläche des zum kapillaren Flüssigkeitstransports befähigten Kanals bildenden Flächen hydrophiliert ist.

3. Analytisches Testelement gemäß Anspruch 2, ***dadurch gekennzeichnet, daß*** die der Aussparung gegenüberliegende, freiliegende Fläche hydrophiliert ist.

4. Analytisches Testelement gemäß einem der Ansprüche 2 oder 3, ***dadurch gekennzeichnet, daß*** die Hydrophilierung durch die Verwendung eines hydrophilen Materials oder durch Beschichtung eines wenig hydrophilen Materials mit einer hydrophilen Schicht erreicht wird.

5. Analytisches Testelement gemäß Anspruch 4, ***dadurch gekennzeichnet, daß*** zur Hydrophilierung eine Schicht aus oxidiertem Aluminium verwendet wird.

6. Analytisches Testelement gemäß einem der Ansprüche 1 bis 5, ***dadurch gekennzeichnet, daß*** das Nachweiselement alle für die Nachweisreaktion des Zielanalyten in der Probe notwendigen Reagenzien sowie gegebenenfalls Hilfsstoffe enthält.

7. Analytisches Testelement gemäß einem der Ansprüche 1 bis 6, ***dadurch gekennzeichnet, daß*** das Nachweiselement als Filter für partikuläre Probenbestandteile wirkt.

8. Analytisches Testelement gemäß einem der Ansprüche 1 bis 7, ***dadurch gekennzeichnet, daß*** der zum kapillaren Flüssigkeitstransport befähigte Kanal zumindest teilweise vom Träger, einer inerten Abdeckung und dem Nachweiselement gebildet wird, wobei Abdeckung und Nachweiselement auf der dem Träger gegenüberliegenden Seite des Kanals liegen und so nebeneinander angeordnet sind, daß die Abdeckung auf der der Probenaufgabeöffnung zugewandten Seite liegt.

9. Analytisches Testelement gemäß Anspruch 8, ***dadurch gekennzeichnet, daß*** *das* Nachweiselement und die Abdeckung Stoß an Stoß nebeneinanderliegend angeordnet sind, so daß der kapillare Flüssigkeitstransport an der Berührunasstelle von Nachweiselement und Abdeckung nicht abreißt.

10. Analytisches Testelement gemäß Anspruch 9, ***dadurch gekennzeichnet, daß*** auf der dem zum kapillaren Flüssigkeitstransport befähigten Kanal hin zugewandten Seite der Abdeckung eine flexible, inerte Folie angebracht ist, die über die gesamte Länge der Abdeckung reicht, den kapillaren Kanal auf der gesamten Breite bedeckt und die zumindest teilweise zwischen den sich gegenüberliegenden Flächen der Abdeckung und des Nachweiselements eingeschlossen ist, so daß der kapillare Flüssigkeitstransport an der Berührungsstelle von Nachweiselement und Abdeckung nicht abreißt.

11. Analytisches Testelement gemäß einem der Ansprüche 1 bis 10, ***dadurch gekennzeichnet, daß*** zwischen Träger und Nachweiselement und gegebenenfalls Abdeckung eine ebenfalls zur Bildung des zum kapillaren Flüssigkeitstransport befähigten Kanals beteiligte Zwischenschicht vorhanden ist.

12. Analytisches Testelement gemäß Anspruch 11, ***dadurch gekennzeichnet, daß*** die Zwischenschicht zusätzlich der Verbindung von Träger und Nachweiselement und gegebenenfalls Abdeckung dient.

13. Verwendung eines analytischen Testelements gemäß einem der Ansprüche 1 bis 12 zur Bestimmung eines Analyten in einer Flüssigkeit.

14. Verfahren zur Bestimmung eines Analyten in einer flüssigen Probe mit Hilfe eines analytischen Testelements gemäß einem der Ansprüche 1 bis 12, wobei die flüssige Probe an der durch die Aussparung unterbrochene Kante der Probenaufgabeöffnung mit dem Testelement kontaktiert wird und durch Kapillarkräfte in den zum kapillaren Flüssigkeitstransport befähigten Kanal transportiert wird, die Probe dabei das Nachweiselement auf der dem Kanal zugewandten Oberfläche benetzt und in dieses eindringt und gegebenenfalls mit den im Nachweiselement enthaltenen Reagenzien eine analytspezifische visuell oder apparativ-optisch, bevorzugt reflexionsphotometrisch beobachtbare Nachweisreaktion eingeht, so daß auf die Anwesenheit und gegebenenfalls die Menge des zu bestimmenden Analyten rückgeschlossen werden kann.

## Claims

1. Analytical test element for the determination of an analyte in a liquid comprising an inert carrier, a detection element and a channel capable of capillary liquid transport which has a sample application opening at one end and a vent opening at the other end of the channel capable of capillary liquid transport, wherein the channel capable of capillary liquid transport being formed at least partially by the carrier and the detection element and extending in the direction of capillary transport from the sample application opening to at least the edge of the detection element that is nearest to the vent opening, *wherein* a notch is located in one of the surfaces forming the channel capable of capillary liquid transport at the edge of the test element forming the sample application opening so that the edge of the test element forming the sample application opening is at least partially discontinuous on one side and the surface opposite to the notch is exposed.

2. Analytical test element as claimed in claim 1, *wherein* at least one of the surfaces forming the inner surface of the channel capable of capillary liquid transport is hydrophilized.

3. Analytical test element as claimed in claim 2, *wherein* the exposed surface opposite to the notch is hydrophilized.

4. Analytical test element as claimed in one of the claims 2 or 3, *wherein* the hydrophilization is achieved by use of a hydrophilic material or by coating a less hydrophilic material with a hydrophilic layer.

5. Analytical test element as claimed in claim 4, *wherein a* layer of oxidized aluminium is used for the hydrophilization.

6. Analytical test element as claimed in one of the claims 1 to 5, *wherein* the detection element contains all necessary reagents for the detection reaction of the target analyte in the sample as well as optionally auxiliary substances.

7. Analytical test element as claimed in one of the claims 1 to 6, *wherein* the detection element acts as a filter for particulate sample components.

8. Analytical test as claimed in one of the claims 1 to 7, *wherein* the channel capable of capillary liquid transport is at least partially formed by the carrier, an inert cover and the detection element wherein the cover and detection element are located on the side of the channel that is opposite to the carrier and are arranged adjacent to one another in such a way that the cover is located on the side facing the sample application opening.

9. Analytical test element as claimed in claim 8, *wherein* the detection element and the cover abut each other so that the capillary liquid transport is not interrupted at the site of contact of detection element and cover.

10. Analytical test element as claimed in claim 9, *wherein* a flexible inert foil is mounted on the side of the cover that faces the channel capable of capillary liquid transport which extends over the entire length of the cover, covers the entire width of the capillary channel and is at least partially enclosed between the opposing surfaces of the cover and detection element so that the capillary liquid transport does not break down at the site of contact between the detection element and cover

11. Analytical test element as claimed in one of the claims 1 to 10, *wherein* an intermediate layer is present between the carrier and detection element and optionally the cover which also participates in the formation of the channel capable of capillary liquid transport.

12. Analytical test element as claimed in claim 11, *wherein* the intermediate layer additionally serves to bond the carrier and detection element and optionally the cover.

13. Use of an analytical test element as claimed in one of the claims 1 to 12 for the determination of an analyte in a liquid.

14. Method for the determination of an analyte in a liquid sample with the aid of an analytical test element as claimed in one of the claims 1 to 12, wherein the liquid sample is contacted with the test element at the edge of the sample application opening which is interrupted by the notch and it is transported by capillary forces into the channel that is capable of capillary liquid transport, in this process the sample wets the surface of the detection element that faces the channel and penetrates into it and optionally an analyte-specific detection reaction occurs with the reagents contained in the detection element which can be observed visually or optically by apparative means preferably by reflection photometry thus enabling conclusions to be drawn about the presence and optionally the amount of the analyte to be determined.

## Revendications

1. Elément d'essai analytique pour la détermination d'un analyte dans un liquide, contenant un support inerte, un élément de décèlement et un canal habilité au transport capillaire de liquides qui possède une ouverture de chargement d'échantillon à une extrémité et une ouverture d'élimination de l'air à l'autre extrémité du canal habilité au transport capillaire de liquides, dans lequel le canal habilité au transport capillaire de liquides est formé au moins en partie par le support et par l'élément de décèlement et qui s'étend dans la direction du transport capillaire depuis l'ouverture de chargement d'échantillon au moins jusqu'au bord de l'élément de décèlement situé le plus près de l'ouverture d'élimination de l'air, **caractérisé en ce qu'**on pratique un évidement en forme d'encoche dans une face formant le canal habilité au transport capillaire de liquides contre le bord de l'élément d'essai formant l'ouverture de chargement d'échantillon, de telle sorte que le bord de l'élément d'essai formant l'ouverture de chargement d'échantillon est interrompu en partie d'un côté et la face opposée à l'évidement est mise à nu.

2. Elément d'essai analytique selon la revendication 1, **caractérisé en ce qu'**au moins une des faces formant la surface interne du canal habilité au transport capillaire de liquides a été rendue hydrophile.

3. Elément d'essai analytique selon la revendication 2, **caractérisé en ce que** la face mise à nu, opposée à l'évidement, a été rendue hydrophile.

4. Elément d'essai analytique selon l'une quelconque des revendications 2 ou 3, **caractérisé en ce qu'**on obtient le caractère hydrophile en utilisant une matière hydrophile ou par enduction d'une matière peu hydrophile avec une couche hydrophile.

5. Elément d'essai analytique selon la revendication 4, **caractérisé en ce qu'**on utilise une couche d'aluminium oxydé pour le traitement rendant hydrophile.

6. Elément d'essai analytique selon l'une quelconque des revendications 1 à 5, **caractérisé en ce que** l'élément de décèlement contient tous les réactifs requis pour la réaction de décèlement de l'analyte cible dans l'échantillon, ainsi que le cas échéant des adjuvants.

7. Elément d'essai analytique selon l'une quelconque des revendications 1 à 6, **caractérisé en ce que** l'élément de décèlement fait office de filtre pour des constituants particulaires de l'échantillon.

8. Elément d'essai analytique selon l'une quelconque des revendications 1 à 7, **caractérisé en ce que** le canal habilité pour le transport capillaire de liquides est formé au moins en partie par le support, par un recouvrement inerte et par l'élément de décèlement, le recouvrement et l'élément de décèlement étant disposés sur le côté du canal opposé au support et étant agencés l'un à côté de l'autre de telle sorte que le recouvrement se trouve sur le côté tourné vers l'ouverture de chargement d'échantillon.

9. Elément d'essai analytique selon la revendication 8, **caractérisé en ce que** l'élément de décèlement et le recouvrement sont agencés de façon à se trouver l'un à côté de l'autre en position bout à bout de telle sorte que le transport capillaire du liquide n'est pas interrompu à l'endroit de contact entre l'élément de décèlement et le recouvrement.

10. Elément d'essai analytique selon la revendication 9, **caractérisé en ce qu'**on applique une feuille flexible et inerte sur le côté du recouvrement tourné vers le canal habilité au transport capillaire de liquides, ladite feuille s'étendant sur toute la longueur de recouvrement, recouvrant le canal capillaire sur toute sa largeur et venant s'insérer au moins en partie entre les faces opposées du recouvrement et de l'élément de décèlement de telle sorte que le transport capillaire du liquide n'est pas interrompu à l'endroit de contact entre l'élément de décèlement et le recouvrement.

11. Elément d'essai analytique selon l'une quelconque des revendications 1 à 10, **caractérisé en ce qu'**on prévoit, entre le support et l'élément de décèlement et, le cas échéant, le recouvrement, une couche intermédiaire qui intervient également pour la formation du canal habilité à un transport capillaire de liquides.

12. Elément d'essai analytique selon la revendication 11, **caractérisé en ce que** la couche intermédiaire a en outre pour fonction de relier le support et l'élément de décèlement et le cas échéant le recouvrement.

13. Utilisation d'un élément d'essai analytique selon l'une quelconque des revendications 1 à 12, pour la détermination d'un analyte dans un liquide.

14. Procédé pour la détermination d'un analyte dans un échantillon liquide à l'aide d'un élément d'essai analytique selon l'une quelconque des revendications 1 à 12, dans lequel l'échantillon liquide est mis en contact avec l'élément d'essai contre le bord de l'ouverture de chargement d'échantillon, interrompu par l'évidement, et est transporté par des forces capillaires dans le canal habilité au transport capillaire de liquides, l'échantillon mouillant en l'occurence l'élément de décèlement sur la surface tournée vers le canal et pénétrant dans ce dernier, et entre le cas échéant en réaction avec les réactifs contenus dans l'élément de décèlement, sous la forme d'une réaction de décèlement spécifique à un analyte qui peut être observé à l'oeil nu ou à l'aide d'un appareil optique, de préférence par photométrie à réflexion, de telle sorte que l'on peut se prononcer sur la présence et le cas échéant sur la quantité de l'analyte à déterminer.
